# EUROPÄISCHE PATENTANMELDUNG

(11) **EP 1 323 828 A2**
(43) Veröffentlichungstag der Anmeldung: **02.07.2003**
(21) Anmeldenummer: 02026847.0
(22) Anmeldetag: 29.11.2002
(51) Int. Cl.: C12P 13/00, C12N 9/88

(54) **Verfahren zur Herstellung von eantiomerenangereicherten Cyanhydrinen unter Verwendung von Acetalen oder Ketalen als Substrate**

(30) Priorität: 27.12.2001 AT 20322001
(71) Anmelder: DSM Fine Chemicals Austria Nfg GmbH & Co KG, 4021 Linz (AT)
(72) Erfinder: Skranc, Wolfgang, 1220 Wien (AT); Pöchlauer, Peter, 4040 Linz (AT); Wirth, Irma, 4470 Enns (AT); Neuhofer, Rudolf, 4210 Mittertreffling (AT); Mayrhofer, Herbert, 4210 Engerwitzdorf (AT)
(74) Vertreter: Lindinger, Ingrid

(57) **Zusammenfassung**

Verfahren zur Herstellung von enantiomerenangereicherten Cyanhydrinen, bei welchem ein Acetal oder Ketal der Formel (I), 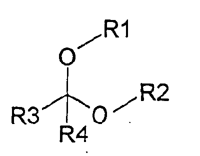 in der R1 und R2 unabhängig voneinander einen gegebenenfalls substituierten C₁-C₂₀-Alkyl-, C₅-C₂₀-Aryl- oder C₅-C₂₀-Heteroarylrest bedeuten,
oder einer der beiden Reste Wasserstoff ist, oder R1 und R2 gemeinsam einen gegebenenfalls substituierten C₂-C₂₀-Alkylenrest bilden,
und R3 und R4 unabhängig voneinander einen gegebenenfalls substituierten C₁-C₂₀-Alkyl-, C₅-C₂₀-Aryl-, C₅-C₂₀-Heteroaryl-, C₇-C₂₀-Alkylaryl-, C₅-C₂₀-Alkylheteroaryl- oder C₅-C₂₀-Aralkylrest oder einen gegebenenfalls substituierten C₅-C₂₀-Heterocyclus oder C₅-C₂₀-Alkylheterocyclus oder gemeinsam einen gegebenenfalls substituierten C₄-C₂₀-Alkylenrest, der ein oder mehrere Heteroatome in der Kette enthalten kann, bedeuten können, oder einer der Reste Wasserstoff bedeutet,
in Gegenwart einer (R)- oder (S)-Hydroxynitrillyase und eines Cyanidgruppendonors im organischen, wässrigen oder Zweiphasensystem oder in Emulsion bei - 5 bis + 40 °C zu den korrespondierenden enantiomerenangereicherten Cyanhydrinen der Formel (II), 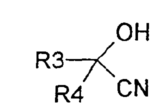 in der R3 und R4 wie oben definiert sind, umgesetzt werden.

## Beschreibung

Cyanhydrine sind zur Synthese von alpha-Hydroxysäuren, alpha-Hydroxyketonen, beta-Aminoalkoholen, die zur Gewinnung biologisch wirksamer Stoffe, z. B. pharmazeutischer Wirkstoffe, Vitamine oder auch pyrethroider Verbindungen Verwendung finden, von Bedeutung.

Die Herstellung dieser Cyanhydrine erfolgt durch Addition von Blausäure an die Carbonylgruppe eines Ketons oder Aldehyds.
Aus der Literatur sind bereits mehrere Verfahrensvarianten bekannt, welche die Herstellung von (R)- und/oder (S)-Cyanhydrinen aus aliphatischen, aromatischen oder heteroaromatischen Aldehyden oder auch aus aliphatischen oder aromatischen Ketonen beschreiben.
So wird in EP-A-0 326 063 ein enzymatisches Verfahren zur Herstellung von optisch aktiven (R)- oder (S)-Cyanhydrinen durch Umsetzung von aliphatischen, aromatischen oder heteroaromatischen Aldehyden oder Ketonen mit Blausäure in Gegenwart von (R)-Oxynitrilase (EC 4.1.2.10) aus Prunus amygdalus oder Oxynitrilase (EC 4.1.2.11) aus Sorghum bicolor beansprucht. Acetale und Ketale werden jedoch nicht beschrieben. EP 0 632 130 beschreibt weiters ein Verfahren, bei welchem aliphatische Aldehyde oder unsymmetrische aliphatische Ketone mit Blausäure und Oxynitrilase aus Hevea brasiliensis stereospezifisch zu (S)-Cyanhydrinen umgesetzt werden.
Acetale und Ketale sind darin nicht enthalten.
In EP 0 927 766 ist ein enzymatisches Verfahren zur Herstellung von optisch aktiven (S)-Cyanhydrinen aus aliphatischen, aromatischen oder heteroaromatischen Aldehyden oder Ketonen in einer Emulsion beschrieben. Acetale und Ketale als Substrate sind hierin nicht beschrieben.
EP 0 528 256 weist darauf hin, dass Hydroxypivaldehyd nur unter Anwendung von stark erhöhter Enzymkonzentration und unter geeigneten Bedingungen zu D-2,4-Dihydroxy-3,3-dimethylbutansäurenitril mit Enantiomerenüberschüssen von bis zu 81,4 % ee umgesetzt werden kann. Bei anderen bisher bekannten Verfahren, die keine stark erhöhten Enzymmengen einsetzen, konnte, wie in EP 0 528 256 beschrieben, kein oder kein genügend hoher Enantiomerenüberschuss erhalten werden. Eine Ursache dafür liegt u. a. auch darin, dass das Substrat Hydroxypivaldehyd nicht stabil ist.

Da viele Carbonylverbindungen, wie etwa Hydroxyaldehyde, welche spontan cyclisieren oder polymerisieren, nicht stabil sind, war es Aufgabe der Erfindung ein Verfahren zu finden, das insbesondere zur Herstellung von optisch aktiven Cyanhydrinen, deren entsprechende Carbonylverbindungen unter bisher bekannten Bedingungen nicht stabil sind, geeignet ist, wobei die Cyanhydrine in guten Ausbeuten und mit einem im Vergleich zu Reaktionen mit freien Aldehyden oder Ketonen als Substrat höheren Enantiomerenüberschuss erhalten werden.

Unerwarteterweise konnte diese Aufgabe durch die Verwendung von Acetalen oder Ketalen als Substrate gelöst werden.

Gegenstand der Erfindung ist demnach ein Verfahren zur Herstellung von enantiomerenangereicherten Cyanhydrinen unter Verwendung von Hydroxynitrillyase (HNL) und einem Cyanidgruppendonor, das dadurch gekennzeichnet ist, dass ein Acetal oder Ketal der Formel (I), 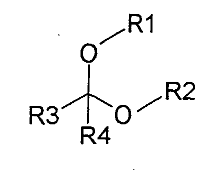 in der R1 und R2 unabhängig voneinander einen gegebenenfalls ein- oder mehrfach substituierten C₁-C₂₀-Alkyl-, C₅-C₂₀-Aryl- oder C₅-C₂₀-Heteroarylrest bedeuten, oder einer der beiden Reste Wasserstoff ist, oder R1 und R2 gemeinsam einen gegebenenfalls ein- oder mehrfach substituierten C₂-C₂₀-Alkylenrest bilden,
und R3 und R4 unabhängig voneinander einen gegebenenfalls ein- oder mehrfach substituierten C₁-C₂₀-Alkyl-, C₅-C₂₀-Aryl-, C₅-C₂₀-Heteroaryl-, C₇-C₂₀-Alkylaryl-, C₅-C₂₀-Alkylheteroaryl- oder C₅-C₂₀-Aralkylrest oder einen gegebenenfalls ein- oder mehrfach substituierten C₅-C₂₀-Heterocyclus oder C₅-C₂₀-Alkylheterocyclus oder gemeinsam einen gegebenenfalls substituierten C₄-C₂₀-Alkylenrest, der ein oder mehrere Heteroatome in der Kette enthalten kann, bedeuten können, oder einer der Reste Wasserstoff bedeutet,
in Gegenwart einer (R)- oder (S)-Hydroxynitrillyase und eines Cyanidgruppendonors im organischen, wässrigen oder Zweiphasensystem oder in Emulsion bei einer Temperatur von - 5 bis + 40 °C zu den korrespondierenden enantiomerenangereicherten Cyanhydrinen der Formel (II), 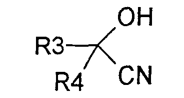 in der R3 und R4 wie oben definiert sind, umgesetzt werden.

Bei dem erfindungsgemäßen Verfahren werden Acetale oder Ketale der Formel (I) als Substrate eingesetzt.
In der Formel (I) können R1 und R2 unabhängig voneinander einen gegebenenfalls ein- oder mehrfach substituierten C₁-C₂₀-Alkyl-, C₅-C₂₀-Aryl- oder C₅-C₂₀-Heteroarylrest bedeuten.
Unter einem C₁-C₂₀-Alkylrest sind dabei gesättigte oder ein- oder mehrfach ungesättigte, lineare, verzweigte oder cyclische, überbrückte, primäre, sekundäre oder tertiäre Hydrocarbonreste zu verstehen. Dies sind beispielsweise Methyl, Ethyl, Propyl, i-Propyl, Propenyl, Butyl, i-Butyl, t-Butyl, Butenyl, Butinyl, Pentyl, Cyclopentyl, i-Pentyl, neo-Pentyl, Pentenyl, Hexyl, i-Hexyl, Cyclohexyl, Cyclohexylmethyl, 3-Methylpentyl, 2,2-Dimethylbutyl, 2,3-Dimethylbutyl, Octyl, Cyclooctyl, Decyl, Cyclodecyl, Dodecyl, Cyclododecyl u.s.w.
Bevorzugt sind dabei C₁-C₁₂-Alkylreste und besonders bevorzugt C₁-C₄-Alkylreste.

Unter Aryl sind bevorzugt C₆-C₂₀-Arylgruppen zu verstehen, wie etwa Phenyl, Biphenyl, Naphthyl, Indenyl, Fluorenyl u.s.w.
Unter Heteroaryl sind bevorzugt cyclische Reste mit 6 bis 20 C-Atomen zu verstehen, die mindestens ein S-, O- oder N-Atom im Ring enthalten.

Die Reste können gegebenenfalls durch einen oder mehrere Substituenten aus der Gruppe Phenyl, C₁-C₆-Alkyl, OH, Halogen oder Sulfoxy substituiert sein.

In diesem Fall werden Vollacetale als Substrate eingesetzt.

Bevorzugt bedeuten R1 und R2 dabei einen Alkylrest.

Werden als Substrate cyclische Acetale verwendet, so bilden R1 und R2 gemeinsam einen C₂-C₂₀-Alkylenrest, der gegebenenfalls durch einen oder mehrere Substituenten aus der Gruppe Phenyl, C₁-C₆-Alkyl, OH, Halogen oder Sulfoxy substituiert sein kann.

Als Substrate eignen sich auch Halbacetale, bei welchen einer der Reste R1 oder R2 Wasserstoff bedeutet.

Besonders bevorzugt bedeuten R1 und R2 unabhängig voneinander einen gesättigten, linearen oder verzweigten C₁-C₄-Alkylrest, oder einer der beiden Reste Wasserstoff, oder bilden R1 und R2 gemeinsam einen C₂-C₆-Alkylenrest, der gegebenenfalls durch einen oder mehrere Substituenten aus der Gruppe C₁-C₄-Alkyl oder OH substituiert sein kann.
R3 und R4 bedeuten in der Formel (I) unabhängig voneinander einen gegebenenfalls ein- oder mehrfach substituierten C₁-C₂₀-Alkyl-, C₅-C₂₀-Aryl-, C₅-C₂₀-Heteroaryl-, C₇-C₂₀-Alkylaryl-, C₅-C₂₀-Alkylheteroaryl- oder C₅-C₂₀-Aralkylrest oder einen gegebenenfalls ein- oder mehrfach substituierten C₅-C₂₀-Heterocyclus oder C₅-C₂₀-Alkylheterocyclus.

Unter C₁-C₂₀-Alkyl sind dabei wiederum gesättigte oder ein- oder mehrfach ungesättigte, lineare, verzweigte oder cyclische, überbrückte, primäre, sekundäre oder tertiäre Hydrocarbonreste zu verstehen, wie etwa Methyl, Ethyl, Propyl, i-Propyl, Propenyl, Butyl, i-Butyl, t-Butyl, Butenyl, Butinyl, Pentyl, Cyclopentyl, i-Pentyl, neo-Pentyl, Pentenyl, Hexyl, i-Hexyl, Cyclohexyl, Cyclohexylmethyl, 3-Methylpentyl, 2,2-Dimethylbutyl, 2,3-Dimethylbutyl, Octyl, Cyclooctyl, Decyl, Cyclodecyl, Dodecyl, Cyclododecyl u.s.w.
Bevorzugt sind dabei C₁-C₁₂-Alkylreste und besonders bevorzugt C₁-C₈-Alkylreste.
Die Alkylgruppe kann gegebenenfalls ein oder mehrfach durch unter den Reaktionsbedingungen inerte Gruppen substituiert sein. Geeignete Substituenten sind beispielsweise gegebenenfalls substituierte Aryl- oder Heteroarylgruppen, wie Phenyl-, Phenoxy- oder Indolylgruppen, Halogen-, Hydroxy-, Hydroxy-C₁-C₅-Alkyl, C₁-C₆-Alkoxy-, Aryloxy-, bevorzugt C₆-C₂₀-Aryloxy-, C₁-C₆-Alkylthio-, Amino-, Alkylamino-, bevorzugt C₁-C₆-Alkylamino-, Arylamino-, bevorzugt C₆-C₂₀-Arylamino-, Ether-, Thioether, Carbonsäureester-, Carbonsäureamid-, Sulfoxid-, Sulfon-, Sulfonsäure, Sulfonsäureester-, Sulfinsäure-, Mercaptan-, Nitro- oder Azidogruppen.

Unter Aryl sind bevorzugt C₆-C₂₀-Arylgruppen zu verstehen, wie etwa Phenyl, Biphenyl, Naphthyl, Indenyl, Fluorenyl u.s.w.

Die Arylgruppe kann dabei gegebenenfalls ein- oder mehrfach substituiert sein. Geeignete Substituenten sind dabei wiederum gegebenenfalls substituierte Aryl- oder Heteroarylgruppen, wie Phenyl-, Phenoxy- oder Indolylgruppen, Halogen-, Hydroxy-, Hydroxy-C₁-C₅-Alkyl, C₁-C₆-Alkoxy-, Aryloxy-, bevorzugt C₆-C₂₀-Aryloxy-, C₁-C₆-Alkylthio-, Amino-, Alkylamino-, bevorzugt C₁-C₆-Alkylamino-, Arylamino-, bevorzugt C₆-C₂₀-Arylamino-, Ether-, Thioether, Carbonsäureester-, Carbonsäureamid-, Sulfoxid-, Sulfon-, Sulfonsäure, Sulfonsäureester-, Sulfinsäure-, Mercaptan-, Nitro- oder Azidogruppen.

Unter Alkaryl oder Alkylaryl sind Alkylgruppen zu verstehen, die einen Arylsubstituenten aufweisen.
Aralkyl oder Arylalkyl bezieht sich auf eine Arylgruppe mit einem Alkylsubstituenten.

Unter Heteroaryl oder Heterocyclus sind cyclische Reste zu verstehen, die mindestens ein S-, O- oder N-Atom im Ring enthalten. Dies sind beispielsweise Furyl, Pyridyl, Pyrimidyl, Thienyl, Isothiazolyl, Imidazolyl, Tetrazolyl, Pyrazinyl, Benzofuranyl, Benzothiophenyl, Chinolyl, Isochinolyl, Benzothienyl, Isobenzofuryl, Pyrazolyl, Indolyl, Isoindolyl, Benzoimidazolyl, Purinyl, Carbazolyl, Oxazolyl, Thiazolyl, Isothiazolyl, 1,2,4-Thiadiazolyl, Isoxazolyl, Pyrrolyl, Chinazolinyl, Pyridazinyl, Phthalazinyl, Morpholinyl, u.s.w.
Funktionelle O- oder N-Gruppen können dabei nötigenfalls geschützt werden.
Die Heteroarylgruppe bzw. der Heterocyclus können dabei gegebenenfalls ein- oder mehrfach durch die bereits oben angeführten Substituenten substituiert sein.

Unter Alkylheteroaryl bzw. Alkylheterocyclus sind dabei Alkylgruppen zu verstehen, die durch eine Heteroarylgruppe bzw. durch einen Heterocyclus substituiert sind.

Bevorzugt bedeuten R3 und R4 einen gesättigten oder ungesättigten, linearen oder verzweigten C₁-C₈-Alkylrest, einen Benzyl- oder einen Phenylrest, wobei die Reste gegebenenfalls ein- oder mehrfach durch OH, Halogen, Phenyl, Carbonsäurederivate, wie Carbonsäureester oder Carbonsäureamide, Amino, C₁-C₆-Alkylamino, C₆-C₂₀-Arylamino, C₁-C₆-Alkoxy, C₆-C₂₀-Aryloxy, oder Nitro substituiert sein können.
R3 und R4 können aber auch gemeinsam einen gegebenenfalls substituierten C₄-C₂₀-Alkylenrest, der ein oder mehrere Heteroatome aus der Gruppe O, N oder S oder eine NR5R6-Gruppe, wobei R5 und R6 unabhängig voneinander H oder C₁-C₆-Alkyl sein können, in der Kette enthalten kann, bedeuten.
Bevorzugt sind C₄-C₇-Alkylenreste, die in Abhängigkeit von der Ringgröße des cyclischen Ketons höchstens zwei Heteroatome in der Alkylkette aufweisen. Der Alkylenrest kann weiters noch, wiederum in Abhängigkeit von der Ringgröße, ein oder zwei Doppelbindungen aufweisen. Der Alkylenrest kann zudem ein oder mehrfach durch die oben angeführten Reste substituiert sein.

Bei den eingesetzten Edukten kann jedoch auch einer der Reste R3 und R4 Wasserstoff bedeuten.

Bei dem erfindungsgemäßen Verfahren werden Acetale oder Ketale der Formel (I), die zum Teil käuflich erwerbbar sind, oder beispielsweise analog Synthesis 1981, 501 - 522 hergestellt werden können, zu enantiomerenangereicherten (R)- und (S)-Cyanhydrinen umgesetzt.

Die entsprechenden Acetale oder Ketale der Formel (I) werden dabei in Gegenwart eines Cyanidgruppendonors mit einer (R)- oder (S)-Hydroxynitrillyase umgesetzt, wobei die Acetale oder Ketale in situ zum korrespondierenden Aldehyd oder Keton und Alkohol gespalten werden und der Aldehyd oder das Keton unter Enzymkatalyse zum entsprechenden enantiomerenangereicherten Cyanhydrin reagiert.

Als Cyanidgruppendonor kommen Blausäure, Alkali-Cyanide oder Cyanhydrine der allgemeinen Formel (III)

R7R8C(OH)(CN),

in Betracht. In der Formel (III) bedeuten R7 und R8 unabhängig voneinander Wasserstoff oder eine unsubstituierte Kohlenwasserstoffgruppe, oder R7 und R8 gemeinsam eine Alkylengruppe mit 4 oder 5 C-Atomen, wobei R7 und R8 nicht gleichzeitig Wasserstoff bedeuten. Die Kohlenwasserstoffgruppen sind aliphatische oder aromatische, bevorzugt aliphatische Gruppen. Bevorzugt bedeuten R7 und R8 Alkylgruppen mit 1 - 6 C-Atomen, besonders bevorzugt ist Acetoricyanhydrin als Cyanidgruppendonor der Formel (III).
Die Herstellung des Cyanidgruppendonors kann nach bekannten Verfahren erfolgen. Cyanhydrine, insbesondere Acetoncyanhydrin, sind auch käuflich zu erwerben.
Bevorzugt wird Blausäure, KCN, NaCN oder Acetoncyanhydrin, besonders bevorzugt Blausäure als Cyanidgruppendonor eingesetzt.

Die Blausäure kann dabei auch erst kurz vor der Reaktion aus einem ihrer Salze, wie etwa NaCN oder KCN, freigesetzt und in Substanz oder in gelöster Form dem Reaktionsgemisch zugegeben werden.

Pro Mol eingesetztem Acetal oder Ketal werden mindestens 1 Mol, bevorzugt 1 bis 5 Mole, besonders bevorzugt 1,2 bis 4 Mole, Cyanidgruppendonor zugegeben.

Die erfindungsgemäße Reaktion findet im organischen, wässrigen oder Zweiphasensystem oder in Emulsion in Anwesenheit einer Hydroxynitrillyase als Katalysator statt.

Dabei wird bei der enantioselektiven Umsetzung im wässrigen System eine wässrige, die entsprechende HNL-enthaltende Lösung oder Pufferlösung verwendet. Beispiele dafür sind Acetatpuffer, Boratpuffer, Phthalatpuffer, Citratpuffer, Phosphatpuffer u.s.w. oder Gemische dieser Pufferlösungen.
Der pH-Wert dieser Lösung liegt dabei bei pH 1,5 bis 5, bevorzugt bei pH 2 bis 4, und besonders bevorzugt bei pH 2,2 bis 3,7.
Der wässrigen Lösung können weiters mit Wasser mischbare oder nicht mischbare Lösungsmitteln, wie etwa Ethanol, DMF, Toluol oder t-Butylmethylether zugegeben werden.

Als organisches Verdünnungsmittel können mit Wasser nicht oder geringfügig mischbare aliphatische oder aromatische Kohlenwasserstoffe, die gegebenenfalls halogeniert sind, Alkohole, Ether oder Ester oder Gemische davon verwendet werden. Bevorzugt werden t-Butylmethylether, Diisopropylether, Dibutylether und Ethylacetat oder deren Gemische eingesetzt.
Die Umsetzung kann jedoch auch in einem Zweiphasensystem oder in Emulsion erfolgen.

Bevorzugt findet die erfindungsgemäße Reaktion in wässriger Lösung in Anwesenheit einer Hydroxynitrillyase als Katalysator statt.

Als HNLs eignen sich sowohl native als auch rekombinante (R)- und (S)-HNLs, die entweder als solche oder immobilisiert vorliegen.

Als (S)-Hydroxynitrillyase kommen native (S)-Hydroxynitrillyasen z. B. aus Maniok und Hevea brasiliensis sowie rekombinante (S)-HNLs in Frage. Bevorzugt wird als native HNL jene aus Hevea brasiliensis verwendet. Geeignete rekombinante (S)-HNL wird beispielsweise aus gentechnisch modifizierten Mikroorganismen, wie etwa Pichia pastoris, E. coli oder Saccharomyces cerevisiae erhalten.
Bevorzugt wird rekombinante (S)-HNL aus Pichia pastoris eingesetzt.

Als (R)-HNL kommen beispielsweise (R)-Hydroxynitrillyasen aus Prunus amygdalus, Prunus laurocerasus oder Prunus serotina oder rekombinante (R)-HNLs in Frage. Bevorzugt wird (R)-Hydroxynitrilase aus Prunus amygdalus oder eine rekombinante (R)-HNL verwendet.

Geeignete (R)- und (S)-HNLs sind beispielsweise aus WO 97/03204, EP 0 969 095; EP 0 951 561, EP 0 927 766, EP 0 632 130, EP 0547 655, EP 0 326 063, WO 01/44487 usw. bekannt.

Bevorzugt wird rekombinante (R)-HNL verwendet.

Pro g Acetal bzw. Ketal werden etwa 10 bis 150 000, bevorzugt 1 200 - 40 000, IU Aktivität Hydroxynitrillyase zugesetzt.

Die Reaktionstemperatur liegt bei etwa - 5 bis + 40 °C, bevorzugt bei etwa 0 bis + 30 °C.

Das erfindungsgemäße Verfahren eignet sich insbesondere zur Herstellung von solchen Cyanhydrinen, deren entsprechende Carbonylverbindungen nicht stabil sind. Dies sind beispielsweise Hydroxyaldehyde, die spontan cyclisieren oder polymerisieren. Die erfindungsgemäß hergestellten Cyanhydrine werden dabei in hohen Ausbeuten und mit einem im Vergleich zu Reaktionen mit freien Aldehyden oder Ketonen als Substrat höheren Enantiomerenüberschuss erhalten.

### Beispiel 1:

5 mL rekombinante (R)-HNL Lösung (240 IU/mL) wurden mit einer Citronensäurelösung auf einen pH-Wert von 2,4 eingestellt. Anschließend wurden 182 µL (1,1 mmol) 1,1-Dimethoxy-2-phenylethan und 0,1 mL (2,3 mmol) Blausäure zugesetzt. Die Reaktionslösung wurde bei Raumtemperatur gerührt. Der Umsatz sowie der Enantiomerenüberschuss des gebildeten (R)-2-Hydroxy-3-phenylpropannitrils wurden mittels GC an einer Cyclodextrinsäule bestimmt.

### Reaktionsverlauf:

| **Stunden** | R)-2-Hydroxy-3-phenylpropannitril | |
|---|---|---|
| | % Umsatz | % ee |
| 3 | 13 | 96,0 |
| 24 | 60 | 95,7 |
| 45,5 | 93 | 96,5 |

### Beispiel 2:

185 µL (1,1 mmol) 3-Chlor-1,1-diethoxypropan wurden zu 5 mL rekombinanter (R)-HNL Lösung (430 IU/mL) mit einem pH-Wert von 2,4 gegeben. Das Gemisch wurde mit 0,1 mL (2,3 mmol) Blausäure versetzt und bei Raumtemperatur gerührt. Nach einer Reaktionszeit von 20 Stunden wurde analysiert und (R)-3-Chlor-2-hydroxybutannitril mit > 98 % ee gefunden. Das Acetal war vollständig abreagiert.

### Beispiel 3:

0,1 mL (2,3 mmol) Blausäure wurden bei 0 °C zu 5 mL rekombinanter (R)-HNL Lösung (800 IU/mL) mit einem pH-Wert von 2,4 gegeben. Anschließend wurde mit 110 mg (0,55 mmol) 4-Hydroxy-β,β,5,5-tetramethyl-1,3-dioxan-2-ethanol (dimerer Hydroxypivalaldehyd) versetzt und bei 0 °C gerührt. Nach einer Reaktionszeit von 22 Stunden waren 95 % zu (R)-2,4-Dihydroxy-3,3-dimethylbutannitril mit 84 % ee umgesetzt.

### Beispiel 4: Vergleichsbeispiel mit Aldehyd als Substrat

110 mg (0,55 mmol) 4-Hydroxy-β,β,5,5-tetramethyl-1,3-dioxan-2-ethanol (dimerer Hydroxypivalaldehyd) wurden bei 110 °C geschmolzen und 40 Minuten bei dieser Temperatur monomerisiert. Das freigesetzte 3-Hydroxy-2,2-dimethylpropanal wurde mit 5 mL rekombinanter (R)-HNL Lösung (3200 IU/mL) mit einem pH-Wert von 2,4 versetzt. Anschließend wurde die Reaktion durch Zugabe von 0,1 mL (2,3 mmol) Blausäure bei 0 °C gestartet. Nach einer Reaktionszeit von 23,5 Stunden bei 0 °C waren 97 % zu (R)-2,4-Dihydroxy-3,3-dimethylbutannitril mit 70 % ee umgesetzt.

## Patentansprüche

1. Verfahren zur Herstellung von enantiomerenangereicherten Cyanhydrinen unter Verwendung von Hydroxynitrillyase und einem Cyanidgruppendonor, das **dadurch gekennzeichnet ist, dass** ein Acetal oder Ketal der Formel (I), 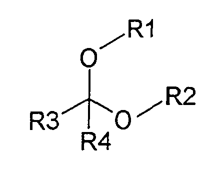 in der R1 und R2 unabhängig voneinander einen gegebenenfalls ein- oder mehrfach substituierten C₁-C₂₀-Alkyl-, C₅-C₂₀-Aryl- oder C₅-C₂₀-Heteroarylrest bedeuten,
oder einer der beiden Reste Wasserstoff ist, oder R1 und R2 gemeinsam einen gegebenenfalls ein- oder mehrfach substituierten C₂-C₂₀-Alkylenrest bilden und R3 und R4 unabhängig voneinander einen gegebenenfalls ein- oder mehrfach substituierten C₁-C₂₀-Alkyl-, C₅-C₂₀-Aryl-, C₅-C₂₀-Heteroaryl-, C₇-C₂₀-Alkylaryl-, C₅-C₂₀-Alkylheteroaryl- oder C₅-C₂₀-Aralkylrest oder einen gegebenenfalls ein- oder mehrfach substituierten C₅-C₂₀-Heterocyclus oder C₅-C₂₀-Alkylheterocyclus oder gemeinsam einen gegebenenfalls substituierten C₄-C₂₀-Alkylenrest, der ein oder mehrere Heteroatome in der Kette enthalten kann, bedeuten können, oder einer der Reste Wasserstoff bedeutet,
in Gegenwart einer (R)- oder (S)-Hydroxynitrillyase und eines Cyanidgruppendonors im organischen, wässrigen oder Zweiphasensystem oder in Emulsion bei einer Temperatur von - 5 bis + 40 °C zu den korrespondierenden enantiomerenangereicherten Cyanhydrinen der Formel (II), 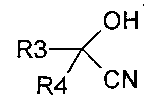 in der R3 und R4 wie oben definiert sind, umgesetzt werden.

2. Verfahren nach Anspruch 1, **dadurch gekennzeichnet, dass** Acetale oder Ketale der Formel (I) als Edukte eingesetzt werden, in der R1 und R2 unabhängig voneinander einen gegebenenfalls ein- oder mehrfach durch OH, Halogen oder Phenyl substituierten, gesättigten, linearen oder verzweigten C₁-C₁₂-Alkylrest bedeuten, oder einer der beiden Reste Wasserstoff bedeutet, oder in der R1 und R2 gemeinsam einen C₂-C₂₀-Alkylenrest bilden, der gegebenenfalls ein- oder mehrfach durch OH, Halogen, Phenyl oder C₁-C₆-Alkyl substituiert sein kann.

3. Verfahren nach Anspruch 1, **dadurch gekennzeichnet, dass** Acetale oder Ketale der Formel (I) als Edukte eingesetzt werden, in der R3 und R4 unabhängig voneinander einen gegebenenfalls ein- oder mehrfach durch OH, Halogen, Phenyl, Carbonsäureester, Carbonsäureamide, Amino, C₁-C₆-Alkylamino, C₆-C₂₀-Arylamino, C₁-C₆-Alkoxy, C₆-C₂₀-Aryloxy oder Nitro substituierten, gesättigten oder ungesättigten, linearen, verzweigten oder cyclischen C₁-C₁₂-Alkyl- oder Phenylrest bedeuten, oder einer der Reste Wasserstoff ist, oder in der R3 und R4 gemeinsam einen gegebenenfalls ein- oder mehrfach durch OH, Halogen, Phenyl, Carbonsäureester, Carbonsäureamide, Amino, C₁-C₆-Alkylamino, C₆-C₂₀-Arylamino, C₁-C₆-Alkoxy, C₁-C₆-Alkyl, C₆-C₂₀-Aryloxy oder Nitro substituierten C₄-C₇-Alkylenrest bedeuten, der ein oder zwei Heteroatome aus der Gruppe O, N oder S oder eine NR5R6-Gruppe, in der R5 und R6 unabhängig voneinander H oder C₁-C₆-Alkyl sein können, in der Kette enthalten kann.

4. Verfahren nach Anspruch 1, **dadurch gekennzeichnet, dass** die enantioselektive Umsetzung im wässrigen System durchgeführt wird, wobei eine die entsprechende Hydroxynitrillyase enthaltende Lösung oder Acetatpuffer- Boratpuffer, Phthalatpuffer-, Citratpuffer- oder Phosphatpufferlösung oder Gemische dieser Pufferlösungen mit einem pH-Wert von 1,5 bis 5 als Reaktionsmedium dient.

5. Verfahren nach Anspruch 4, **dadurch gekennzeichnet, dass** dem wässrigen System ein mit Wasser mischbares oder nicht mischbares Lösungsmittel aus der Gruppe der Ethanol, t-Butylmethylether, Diisopropylether, Dibutylether, Dimethylformamid, Toluol oder Ethylacetat oder Gemische davon zugesetzt wird.

6. Verfahren nach Anspruch 1, **dadurch gekennzeichnet, dass** als Hydroxynitrillyase native oder rekombinante (R)- und (S)-Hydroxynitrillyasen eingesetzt werden, die entweder als solche oder immobilisiert vorliegen.

7. Verfahren nach Anspruch 6, **dadurch gekennzeichnet, dass** als Hydroxynitrillyase native (S)-Hydroxynitrillyasen aus Maniok und Hevea brasiliensis, rekombinante (S)-Hydroxynitrillyasen aus gentechnisch modifizierten Mikroorganismen aus der Gruppe Pichia pastoris, E. coli oder Saccharomyces cerevisiae, native (R)-Hydroxynitrillyasen aus Prunus amygdalus, Prunus laurocerasus oder Prunus serotina oder rekombinante (R)-Hydroxynitrillyasen eingesetzt werden.

8. Verfahren nach Anspruch 6, **dadurch gekennzeichnet, dass** rekombinante (R)-Hydroxynitrillyasen eingesetzt werden.

9. Verfahren nach Anspruch 1, **dadurch gekennzeichnet, dass** als Cyanidgruppendonor Blausäure, Alkalicyanide oder Cyanhydrine der allgemeinen Formel (III),
R7R8C(OH)(CN)
in der R7 und R8 unabhängig voneinander Wasserstoff oder eine unsubstituierte Kohienwasserstoffgruppe bedeuten, oder R7 und R8 gemeinsam eine Alkylengruppe mit 4 oder 5 C-Atomen, wobei R7 und R8 nicht gleichzeitig Wasserstoff bedeuten, bilden.

10. Vertahren nach Anspruch 9, **dadurch gekennzeichnet, dass** als Cyanidgruppendonor Blausäure, KCN, NaCN oder Acetoncyanhydrin eingesetzt wird.
